# FASCICULE DE BREVET EUROPEEN

(11) **EP 2 748 306 B1**
(45) Date de publication et mention de la délivrance du brevet: **27.07.2016**
(21) Numéro de dépôt: 12758577.6
(22) Date de dépôt: 23.08.2012
(51) Int. Cl.: C12N 5/0775, C12N 5/071, A01N 1/02

(54) **UTILISATION D'HEMOGLOBINE D'ANNELIDES POUR MAINTENIR DES CELLULES SOUCHES A L'ETAT INDIFFERENCIE**
VERWENDUNG VON RINGELWURM-HÄMOGLOBIN ZUR BEWAHRUNG VON STAMMZELLEN IN UNDIFFERENZIERTEM ZUSTAND
USE OF ANNELID HAEMOGLOBIN FOR MAINTAINING STEM CELLS IN THE UNDIFFERENTIATED STATE

(30) Priorité: 26.08.2011 FR 1157567
(43) Date de publication de la demande: 02.07.2014
(73) Titulaire: Hemarina, 29600 Morlaix (FR)
(72) Inventeur: ZAL, Franck, F-29600 Ploujean-Morlaix (FR)
(74) Mandataire: Cabinet Plasseraud
(86) Numéro de dépôt international: PCT/FR2012/051925
(87) Numéro de publication internationale: WO 2013/030496

(56) Documents cités:
- WO-A2-2008/143884
- FR-A1- 2 919 785
- CHEN HSIN-FU ET AL: "Hypoxic Culture Maintains Self-Renewal and Enhances Embryoid Body Formation of Human Embryonic Stem Cells", TISSUE ENGINEERING PART A, vol. 16, no. 9, septembre 2010 (2010-09), pages 2901-2913, XP002671810, ISSN: 1937-3341

## Description

La présente invention a trait à l'utilisation d'au moins une globine, un protomère de globine ou une hémoglobine extracellulaire d'Annélides, pour maintenir des cellules souches à l'état indifférencié.

Les cellules souches constituent aujourd'hui un potentiel thérapeutique intéressant, notamment du point de vue de la médecine régénérative ou thérapeutique. Elles regroupent différentes catégories cellulaires spécifiques:
- les cellules souches adultes : les premières découvertes ont été les cellules sanguines. Elles sont très utilisées après une chimiothérapie, qui détruit les cellules de la moelle osseuse. Une greffe autologue (du patient à lui-même) permet de produire plus rapidement des cellules sanguines, sans risque de rejet. Tous les tissus et tous les organes contiennent des cellules souches adultes, mais elles sont rares et difficiles à purifier ;
- les cellules souches périnatales : elles sont contenues dans le cordon, le placenta et le sang qui s'y trouve. On peut citer les cellules souches sanguines, qui sont utilisables pour des greffes, mais doivent être compatibles d'un point de vue immunologique ;
- les cellules souches mésenchymateuses : elles peuvent être aussi bien périnatales (contenues dans le cordon et le placenta) qu'adultes. Elles proviennent alors principalement de la moelle osseuse et du tissu adipeux ;
- les cellules souches embryonnaires : ces cellules proviennent d'embryons dits surnuméraires, et sont particulièrement intéressantes pour la recherche car elles sont complètement indifférenciées et peuvent se spécialiser pour l'ensemble des tissus humains. Il existe bien sûr des applications médicales (pallier aux déficiences de certains organes) mais aussi pharmacologiques ; et enfin
- les cellules reprogrammées, également appelées « cellules souches pluripotentes induites » ou « iPS » : en introduisant 4 gènes capables de complètement la reprogrammer, les chercheurs ont transformé une cellule différenciée adulte de la peau en cellule souche pluripotente. Cette technique a été employée sur des souris atteintes de drépanocytose. Les globules rouges malades ont été corrigés par génie génétique, dédifférenciés puis réinjectés à la souris. Ainsi une guérison a-t-elle pu être obtenue à partir de cellules de la souris malade elle-même.

Afin de pouvoir utiliser ces cellules souches en thérapie et développer leurs applications, il est nécessaire de les conserver, le temps voulu, à l'état indifférencié. En effet, celles-ci se différencient très rapidement en un type cellulaire donné, ce qui les rend inutilisables pour (ré)orienter leur différentiation vers un type cellulaire différent, ou pour effectuer des travaux de recherche fondamentale sur ces cellules.

Il existe donc un besoin pour maintenir des cellules souches, notamment animales, plus particulièrement humaines, à l'état indifférencié, afin d'enclencher le processus de différenciation à l'instant souhaité.

Les inventeurs ont maintenant découvert que, de façon surprenante, l'hémoglobine extracellulaire de la famille des *Nereididae,* lorsqu'elle est additionnée à des cellules souches, notamment humaines, permet de maintenir ces dernières à l'état indifférencié, tout en conservant leur viabilité.

La présente invention concerne ainsi l'utilisation d'au moins une globine, un protomère de globine ou une hémoglobine extracellulaire de la famille des *Nereididae,* pour maintenir des cellules souches choisies parmi les cellules progénitrices animales et les cellules souches mésenchymateuses animales, à l'état indifférencié. Les cellules souches peuvent être murines ou humaines, de préférence elles sont humaines.

L'hémoglobine extracellulaire d'Annélides est présente chez les trois classes d'Annélides : les Polychètes, les Oligochètes et les Achètes. On parle d'hémoglobine extracellulaire car elle est naturellement non contenue dans une cellule, et peut donc circuler librement dans le système sanguin sans modification chimique pour la stabiliser ou la rendre fonctionnelle.

L'hémoglobine extracellulaire d'Annélides est un biopolymère géant de poids moléculaire compris entre 2000 et 4000 kDa, constitué d'environ 200 chaînes polypeptidiques comprises entre 4 et 12 types différents que l'on regroupe généralement en deux catégories.

La première catégorie, comptant 144 à 192 éléments, regroupe les chaînes polypeptidiques dites "fonctionnelles" qui portent un site actif de type hème, et sont capables de lier réversiblement l'oxygène ; ce sont des chaînes de type globine dont les masses sont comprises entre 15 et 18 kDa et qui sont très similaires aux chaînes de type α et β de vertébrés.

La deuxième catégorie, comptant 36 à 42 éléments, regroupe les chaînes polypeptidiques dites de "structure" ou « linkers » possédant peu ou pas de site actif mais permettant l'assemblage des sous-unités appelées douzièmes ou protomères.

Chaque molécule d'hémoglobine est constituée de deux hexagones superposés que l'on a nommés bicouche hexagonale (hexagonal bilayer) et chaque hexagone est lui-même formé par l'assemblage de six sous-unités (ou "douzièmes" ou « protomères ») en forme de goutte d'eau. La molécule native est formée de douze de ces sous-unités (dodécamère ou protomère). Chaque sous-unité a une masse moléculaire comprise entre 200 et 250 kDa, et constitue l'unité fonctionnelle de la molécule native.

L'hémoglobine extracellulaire d'Annélides est choisie parmi les hémoglobines extracellulaires de la famille des *Nereididae.* Encore plus préférentiellement, l'hémoglobine extracellulaire d'Annélides est l'hémoglobine extracellulaire de *Nereis sp,* plus préférentiellement l'hémoglobine extracellulaire de *Nereis virens.*

Selon l'invention, le protomère de globine de l'hémoglobine extracellulaire d'Annélides constitue l'unité fonctionnelle de l'hémoglobine native, comme indiqué ci-dessus.

Enfin, la chaîne de globine de l'hémoglobine extracellulaire d'Annélides peut notamment être choisie parmi les chaînes de globine de type Ax et/ou Bx d'hémoglobine extracellulaire d'Annélides.

L'hémoglobine extracellulaire d'Annélides et ses protomères de globine ont une activité superoxide dismutase (SOD) intrinsèque, et ne nécessitent, par conséquent, aucun antioxydant pour fonctionner, contrairement à l'utilisation d'une hémoglobine de mammifères, pour laquelle les molécules antioxydantes sont contenues à l'intérieur du globule rouge et ne sont pas liées à l'hémoglobine. D'autre part, l'hémoglobine extracellulaire d'Annélides, ses protomères de globine et/ou ses globines ne nécessitent pas de cofacteur pour fonctionner, contrairement à l'hémoglobine de mammifère, notamment humaine. Enfin, l'hémoglobine extracellulaire d'Annélides, ses protomères de globine et/ou ses globines ne possédant pas de typage sanguin, ils permettent d'éviter tout problème de réaction immunologique.

L'hémoglobine extracellulaire d'Annélides, ses protomères de globine et/ou ses globines peuvent être natifs ou recombinants.

Selon l'invention, de préférence, la globine, le protomère de globine ou l'hémoglobine extracellulaire d'Annélides est présente dans une composition comprenant une solution tampon. Dans ce cas, de préférence, la globine, le protomère de globine ou l'hémoglobine extracellulaire d'Annélides est présent dans la composition à une concentration comprise entre 0.001 et 10 mg/ml, de préférence entre 0.20 et 1.50 mg/ml, de préférence entre 0.25 et 1.25 mg/ml.

Ladite solution tampon créée un environnement salin adéquat pour l'hémoglobine, ses protomères et ses globines, et permet ainsi le maintien de la structure quaternaire, et donc de la fonctionnalité de cette molécule. Grâce à la solution tampon, l'hémoglobine, ses protomères et ses globines sont capables d'assurer leur fonction d'oxygénation.

La solution tampon selon l'invention est une solution aqueuse comprenant des sels, de préférence des ions chlorure, sodium, calcium, magnésium et potassium, et confère à la composition selon l'invention un pH compris entre 6.5 et 7.6 ; sa formulation est similaire à celle d'un liquide physiologiquement injectable. Dans ces conditions, l'hémoglobine extracellulaire d'Annélides, ses protomères de globine et ses globines restent fonctionnels.
Dans la présente description, le pH s'entend à température ambiante (25°C), sauf mention contraire.

De préférence, la solution tampon est une solution aqueuse comprenant du chlorure de sodium, du chlorure de calcium, du chlorure de magnésium, du chlorure de potassium, ainsi que du sodium gluconate et du sodium acétate, et a un pH compris entre 6.5 et 7.6, de préférence égal à 7,1 ± 0,5, de préférence d'environ 7.35. Plus préférentiellement, la solution tampon est une solution aqueuse comprenant 90 mM de NaCl, 23 Mm de Na-gluconate, 2,5 mM de CaCl₂, 27 mM de Na-acétate, 1,5 mM de MgCl₂, 5 mM de KCI, et a un pH de 7,1 ± 0,5, pouvant contenir entre 0 et 100 mM d'antioxydant de type acide ascorbique et/ou glutathion réduit.

De préférence, les cellules souches maintenues à l'état indifférencié selon l'invention sont choisies parmi les cellules souches pluripotentes induites (iPS), les cellules souches embryonnaires, les cellules souches périnatales, les cellules souches adultes et les cellules souches mésenchymateuses. Lesdites cellules souches sont de préférence animales, encore plus préférentiellement humaines.

La présente invention se rapporte également à un procédé de conservation de cellules souches choisies parmi les cellules progénitrices animales et les cellules souches mésenchymateuses animales, à l'état indifférencié, de préférence humaines, comprenant une étape de mélange de cellules souches avec une solution tampon comprenant au moins une globine, un protomère de globine ou une hémoglobine extracellulaire de la famille des *Nereididae.* Les globine, protomère de globine et hémoglobine extracellulaire de la famille des *Nereididae* sont de préférence tels que décrits ci-dessus.

L'hémoglobine extracellulaire d'Annélides selon l'invention, de préférence présente dans une solution tampon, peut être ajoutée directement aux cellules souches ou au milieu les comprenant. De préférence, lesdites cellules souches sont maintenues pendant une durée d'environ 1 à 15 jours en contact avec l'hémoglobine extracellulaire d'Annélides, voire plus longtemps en fonction de l'application envisagée. Ensuite, si l'hémoglobine extracellulaire d'Annélides est éliminée, par exemple par filtration ou pipetage, les cellules souches peuvent à nouveau se différencier.

L'invention est décrite plus en détail dans les exemples suivants. Ces exemples sont fournis à des fins d'illustration uniquement, et ne sont pas limitatifs.

Les figures illustratives sont les suivantes :
Figure 1 : Détermination du pourcentage d'occupation des boîtes CFE par les clones après fixation et coloration des clones obtenus après 13 jours de culture par le logiciel « Image J » pour la population enrichie en cellules souches. Les résultats sont les moyennes des triplicats par condition.
Figure 2 : Résultats du comptage du nombre de clones présents dans les boîtes CFE après fixation et coloration des clones obtenus après 13 jours de culture pour la population enrichie en cellules souches. Les résultats sont les moyennes des triplicats par condition.

### Exemples

### Matériel et méthodes

### Actif

L'hémoglobine extracellulaire d'Annélides, ici de *Nereis virens,* en solution dans une solution tampon comprenant 90 mM de NaCl, 23 Mm de Na-gluconate, 2,5 mM de CaCl₂, 27 mM de Na-acétate, 1,5 mM de MgCl₂, 5 mM de KCI, et a un pH de 7,1 ± 0,5, pouvant contenir entre 0 et 100 mM d'antioxydant de type acide ascorbique et/ou glutathion réduit, est utilisée aux concentrations testées de 0.25, 0.75 et 1.25 mg/mL. Cette hémoglobine est disponible sous la référence Hemarina-M201 par la société Hemarina.

### Test de clonoqénicité CFE (2D)

Principe : Le test d'efficacité clonogénique CFE permet d'évaluer la fréquence de kératinocytes capables de générer un clone cellulaire au sein d'une population donnée. Il est considéré qu'un fort potentiel clonogénique est associé aux cellules souches/progénitrices, alors que des kératinocytes différenciés perdent cette potentialité. Ce test de culture consiste à ensemencer des kératinocytes à faible densité (de 100 à 1000 cellules par boîte), de manière à obtenir des clones bien individualisés, aisément comptables et faciles à distinguer d'un point de vue qualitatif. La culture est réalisée en milieu contenant du sérum sur couche nourricière de fibroblastes irradiés.

Protocole: l'hémoglobine extracellulaire de *Nereis virens* (Hemarina-M201) a été testée indépendamment sur différentes populations cellulaires :
- Test sur banque de kératinocytes tout venant pré-amplifiés : immédiatement après décongélation, les cellules ont été ensemencées à raison de 100 cellules par boîte de Pétri en conditions CFE en présence d' Hemarina-M201 ou non (témoin) dans le milieu de culture.
- Test sur populations cellulaires fraîchement extraites à partir de d'une plastie mammaire cutanée et ensuite enrichies ou déplétées en cellules souches et progéniteurs épidermiques : A J0, les cellules ont été ensemencées à raison de 1000 cellules par boîte de Pétri en conditions CFE en présence ou non (témoin) d'Hemarina-M201 dans le milieu de culture.
A J3 et J8 de culture, les milieux ont été changés en présence ou non (témoin) d'Hemarina-M201 dans le milieu de culture. La culture a été poursuivie jusqu'à J13 puis arrêtée, fixée et les clones cellulaires ont été colorés et analysés. Chaque condition a été testée en triplicata.

### Résultats

### Evaluation d'Hemarina-M201 sur population de kératinocytes tout-venants à partir de deux banques:

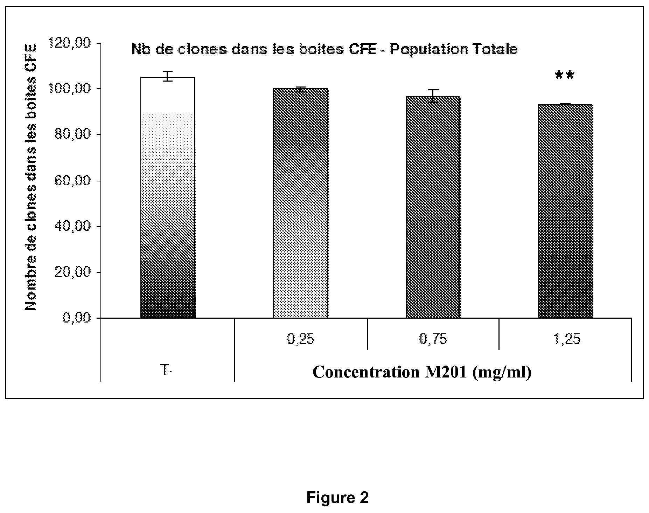

Pour la première banque, le nombre de clones présents dans les boîtes CFE après 13 jours de culture n'est pas significativement différent entre les conditions témoin, Hemarina-M201 à 0.25 mg/ml, Hemarina-M201 à 0.75 mg/ml et Hemarina-M201 à 1.25 mg/ml (Figure 2). Hemarina-M201 n'influe donc pas sur la viabilité desdits clones.

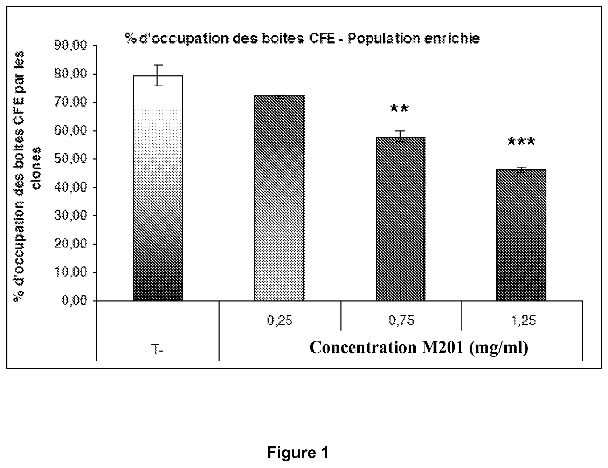

En revanche, le pourcentage d'occupation des boîtes CFE par les clones après 13 jours de culture est significativement différent entre la condition témoin et les conditions Hemarina-M201 à 0.25 mg/ml, Hemarina-M201 à 0.75 mg/ml et Hemarina-M201 à 1.25 mg/ml : il est inférieur pour les conditions avec Hemarina-M201, de façon dose dépendante (Figure 1). Ce pourcentage d'occupation des boites CFE est d'autant plus faible que la concentration en Hemarina-M201 est forte.

Hemarina-M201 influe donc sur la différenciation cellulaire. Le nombre de clones viables en présence d'Hemarina-M201 est le même aux différents temps qu'au temps T0, mais en absence d'Hemarina-M201, les cellules se sont différencié comme le montre la surface d'occupation des boites qui augmente.

Les résultats obtenus avec l'autre banque sont similaires à ceux obtenus avec la première banque.

En conclusion, quelle que soit la banque cellulaire étudiée, le nombre de clones cellulaires obtenus ne semble pas évoluer drastiquement étant donné les écarts types.
Ces résultats indiquent que l'hémoglobine Hemarina-M201 empêche la différentiation cellulaire des cellules souches de kératinocytes tout-venants issus des banques, tout en conservant leur viabilité.

### Evaluation d'Hemarina-M201 sur des populations de kératinocytes fraîchement extraits, enrichies ou déplétées en cellules souches et cellules progénitrices :

### Population enrichie en cellules souches et cellules progénitrices :

Le pourcentage d'occupation des boîtes CFE par les clones après 13 jours de culture est significativement inférieur pour les conditions Hemarina-M201 à 0.75 mg/ml et Hemarina-M201 à 1.25 mg/ml, comparé au témoin et à Hemarina-M201 à 0.25 mg/ml.

### Population tout-venant de kératinocytes fraîchement isolés :

Le nombre de clones présents dans les boîtes CFE après 13 jours de culture n'est pas significativement différent entre les conditions témoin, Hemarina-M201 à 0.25 mg/ml et Hemarina-M201 à 0.75 mg/ml, mais est significativement supérieur à la condition Hemarina-M201 à 1.25 mg/ml.

Le pourcentage d'occupation des boîtes CFE par les clones après 13 jours de culture est significativement inférieur pour les conditions Hemarina-M201 à 0.25, 0.75 et 1.25 mg/ml, et ce de manière dose dépendante, comparé au témoin.

### Population déplétée en cellules souches et cellules progénitrices :

Le nombre de clones présents dans les boîtes CFE après 13 jours de culture n'est pas significativement différent entre les conditions témoin et Hemarina-M201 à 0.25 mg/ml. En revanche, les conditions Hemarina-M201 à 0.75 et 1.25 mg/ml ont des nombres de clones significativement inférieurs, de façon dose dépendante.

Le pourcentage d'occupation des boîtes CFE par les clones après 13 jours de culture est significativement inférieur pour les conditions Hemarina-M201 à 0.25, 0.75 et 1.25 mg/ml, et ce de manière dose dépendante, comparé au témoin.

En conclusion, quelle que soit la population cellulaire étudiée (population enrichie, tout-venant ou déplétée en cellules souches et cellules progénitrices), l'hémoglobine Hemarina-M201 bloque le potentiel de différentiation des cellules souches donnant des kératinocytes. Le nombre de clones n'évolue pas en présence d'Hemarina-M201 et leur viabilité demeure intacte.

### Conclusion

Ces études montrent que l'hémoglobine Hemarina-M201 a une action sur le potentiel clonogénique des populations cellulaires testées. Hemarina-M201 empêche la différentiation des cellules souches, tout en conservant leur viabilité.

## Revendications

1. Utilisation *in vitro* d'au moins une globine, un protomère de globine ou une hémoglobine extracellulaire de la famille des *Nereididae,* pour maintenir des cellules souches choisies parmi les cellules progénitrices animales et les cellules souches mésenchymateuses animales, à l'état indifférencié.

2. Utilisation selon la revendication 1, **caractérisée en ce que** la globine, le protomère de globine ou l'hémoglobine extracellulaire de la famille des *Nereididae* est présente dans une composition comprenant une solution tampon.

3. Utilisation selon l'une des revendications 1 ou 2, **caractérisée en ce que** l'hémoglobine extracellulaire de la famille des *Nereididae* est l'hémoglobine extracellulaire de *Nereis sp.*

4. Utilisation selon l'une des revendications 1 à 3, **caractérisée en ce que** l'hémoglobine extracellulaire de la famille des *Nereididae* est l'hémoglobine extracellulaire de *Nereis virens.*

5. Utilisation selon l'une des revendications 2 à 4, **caractérisée en ce que** la globine, le protomère de globine ou l'hémoglobine extracellulaire de la famille des *Nereididae* est présent dans la composition à une concentration comprise entre 0.001 et 10 mg/ml, de préférence entre 0.20 et 1.50 mg/ml.

6. Utilisation selon l'une des revendications 1 à 5, **caractérisée en ce que** les cellules souches sont humaines.

7. Procédé de conservation de cellules souches à l'état indifférencié, comprenant une étape de mélange de cellules souches choisies parmi les cellules progénitrices animales et les cellules souches mésenchymateuses animales, avec une solution tampon comprenant au moins une globine, un protomère de globine ou une hémoglobine extracellulaire de la famille des *Nereididae.*

8. Utilisation selon l'une des revendications 2 à 6 ou procédé selon la revendication 7, **caractérisé en ce que** la solution tampon comprend du chlorure de sodium, du chlorure de calcium, du chlorure de magnésium, du chlorure de potassium, ainsi que du sodium gluconate et du sodium acétate, et a un pH compris entre 6.5 et 7.6, de préférence égal à 7,1 ± 0,5, de préférence d'environ 7.35.

## Patentansprüche

1. In-vitro-Anwendung
wenigstens eines Globins, eines Globin-Protomers oder eines extrazellulären Hämoglobins der Familie der *Nereididae* zur Bewahrung von Stammzellen, die aus tierischen Vorläuferzellen und tierischen mesenchymalen Stammzellen im undifferenzierten Zustand gewählt wurden.

2. Anwendung nach Anspruch 1, **dadurch gekennzeichnet, dass** das Globin, Globin-Protomer oder extrazelluläre Hämoglobin der Familie der *Nereididae* in einer Zusammensetzung präsentiert wird, die eine Pufferlösung umfasst.

3. Anwendung nach einem der Ansprüche 1 oder 2, **dadurch gekennzeichnet, dass** das extrazelluläre Hämoglobin der Familie der *Nereididae* extrazelluläres Hämoglobin von *Nereis sp.* ist.

4. Anwendung nach einem der Ansprüche 1 bis 3, **dadurch gekennzeichnet, dass** das extrazelluläre Hämoglobin der Familie der *Nereididae* extrazelluläres Hämoglobin von *Nereis virens* ist.

5. Anwendung nach einem der Ansprüche 2 bis 4, **dadurch gekennzeichnet, dass** das Globin, Globin-Protomer oder extrazelluläre Hämoglobin der Familie der *Nereididae* in einer Zusammensetzung mit einer Konzentration zwischen 0,001 und 10 mg/ml, vorzugsweise zwischen 0,20 und 1,50 mg/ml vorliegt.

6. Anwendung nach einem der Ansprüche 1 bis 5, **dadurch gekennzeichnet, dass** es sich um menschliche Stammzellen handelt.

7. Verfahren zum Konservieren von Stammzellen in undifferenziertem Zustand, welches einen Schritt des Mischens von Stammzellen umfasst, die gewählt sind aus tierischen Vorläuferzellen und tierischen mesenchymalen Stammzellen, mit einer Pufferlösung, welche wenigstens ein Globin, Globin-Protomer oder extrazelluläres Hämoglobin der Familie der *Nereididae* umfasst.

8. Anwendung nach einem der Ansprüche 2 bis 6 oder Verfahren nach Anspruch 7,
**dadurch gekennzeichnet, dass** die Pufferlösung Natriumchlorid, Kalziumchlorid, Magnesiumchlorid, Kaliumchlorid sowie Natriumgluconat oder Natriumacetat umfasst und einen pH-Wert zwischen 6,5 und 7,6, vorzugsweise gleich 7,1 +/- 0,5, vorzugsweise gleich ungefähr 7,35 aufweist.

## Claims

1. *In vitro* use of at least one globin, a globin protomer or an extracellular haemoglobin of the *Nereididae* family, in order to maintain stem cells chosen from animal progenitor cells and animal mesenchymatous stem cells, in the undifferentiated state.

2. Use according to claim 1, **characterised in that** the globin, the globin protomer or the extracellular haemoglobin of the *Nereididae* is present in a composition comprising a buffer solution.

3. Use according to one of claims 1 or 2, **characterised in that** the extracellular haemoglobin of the *Nereididae* family is the extracellular haemoglobin of *Nereis sp.*

4. Use according to one of claims 1 to 3, **characterised in that** the extracellular haemoglobin of the *Nereididae* family is the extracellular haemoglobin of *Nereis virens.*

5. Use according to one of claims 2 to 4, **characterised in that** the globin, the globin protomer or the extracellular haemoglobin of the *Nereididae* family is present in the composition at a concentration of between 0.001 and 10 mg/ml, preferably between 0.20 and 1.50 mg/ml.

6. Use according to one of claims 1 to 5, **characterised in that** the stem cells are human.

7. Method for preserving stem cells in the undifferentiated state, comprising a step of mixing stem cells chosen from animal progenitor cells and animal mesenchymatous stem cells, with a buffer solution comprising at least one globin, a globin protomer or an extracellular haemoglobin of the *Nereididae* family.

8. Use according to one of claims 2 to 6 or method according to claim 7, **characterised in that** the buffer solution comprises sodium chloride, calcium chloride, magnesium chloride and potassium chloride, as well as sodium gluconate and sodium acetate, and has a pH of between 6.5 and 7.6, preferably equal to 7.1 ± 0.5, preferably approximately 7.35.
